Europäisches Patentamt

European Patent Office

Office européen des brevets

(19)

(11) Numéro de publication : **0 121 473**
**B1**

(12) **FASCICULE DE BREVET EUROPÉEN**

(45) Date de publication du fascicule du brevet :
27.07.88

(51) Int. Cl.⁴ : **A 61 F   2/24**

(21) Numéro de dépôt : 84400631.2

(22) Date de dépôt : 29.03.84

(54) **Valve cardiaque artificielle à ouverture active.**

(30) Priorité : 30.03.83 FR 8305274

(43) Date de publication de la demande :
10.10.84 Bulletin 84/41

(45) Mention de la délivrance du brevet :
27.07.88 Bulletin 88/30

(84) Etats contractants désignés :
AT BE CH DE FR GB IT LI LU NL SE

(56) Documents cités :
FR-A- 2 331 997
US-A- 3 370 305
US-A- 3 859 668
US-A- 3 974 854

(73) Titulaire : **UNIVERSITE PIERRE ET MARIE CURIE PARIS VI**
**4, place Jussieu**
**F-75230 Paris Cedex 05 (FR)**

(72) Inventeur : **Carpentier, Alain**
**96 rue Didot**
**F-75014 Paris (FR)**

(74) Mandataire : **Portal, Gérard et al**
**Cabinet Beau de Loménie 55, rue d'Amsterdam**
**F-75008 Paris (FR)**

## Description

La présente invention concerne une valve cardiaque artificielle destinée à être implantée chez l'homme.

Il est connu (CARDIAC VALVES PROSTESIS de E.A. LEFRAK et A. STARR - Appleton-Century Crofts) de remplacer les valves cardiaques de l'homme par des prothèses qui, toutes, présentent un dispositif de clapet laissant s'écouler le sang dans un sens et l'empêchant de refluer dans l'autre. Ces valves comportent soit une bille de plastique jouant dans une cage (valve de Starr) et dégageant ou obstruant alternativement un siège métallique fixé au coeur, soit un disque se déplaçant parallèlement dans une cage suivant les mêmes modalités que plus haut (valve de Beall), soit un disque oscillant maintenu par deux griffes (valve de Björk).

Ces prothèses risquent de provoquer la coagulation du sang, ce qui peut bloquer le mécanisme de la prothèse et/ou produire des embolies. Ce risque est principalement lié à la configuration même de ces prothèses qui produisent des turbulences autour de la bille ou du disque d'occlusion de la valve.

Il est connu par ailleurs un autre mécanisme dit « valve papillon » constitué par un disque en élastomère fixé à un axe central (Goot). Ce disque repose pendant le temps de fermeture sur des picots ou dents radialement disposées, implantées sur le corps de valve. En raison de l'existence d'un axe central fixe et de picots ou dents créant des turbulences, les risques de coagulation sont également importants et cette prothèse a été abandonnée.

Le document US-A-3 859 668 décrit une valve cardiaque comportant un disque mobile dans un siège ; le disque obturant partiellement le siège en position de fermeture. Cette valve comporte un dispositif permettant d'éviter le retour du sang et d'assurer une ouverture déterminée.

Dans la demande de brevet français 75/36074 (FR-A- 2 331 997) on a décrit une valve artificielle comportant :

a) un anneau sensiblement circulaire comportant des moyens de sutures de type connu en soi,

b) deux clapets demi-circulaire mobiles reposant sur une rampe intérieure de cet anneau,

c) un système de charnière à double déplacement permettant auxdits clapets d'effectuer des mouvements de rotation puis de translation dégageant le système charnière, dispositif qui par le « lavage » qu'il autorise permet de diminuer les risques de coagulation.

Quelles que soient les qualités des diverses valves artificielles actuellement connues, elles gardent deux inconvénients majeurs :

1. il s'agit de valves à ouverture passive s'ouvrant uniquement sous des contraintes mécaniques (gradients de pression) et non pas de valves à ouverture active qui s'ouvrent d'elles-mêmes comme les valves naturelles, dites mitrales, au temps approprié en l'absence de tout gradient de pression permettant ainsi un meilleur remplissage des ventricules ;

2. il s'agit de valves bruyantes, produisant lors du temps de fermeture un bruit métallique audible aussi bien par le malade que par l'entourage.

La présente invention qui vise notamment à supprimer les difficultés mentionnées ci-dessus pour les diverses valves artificielles permet l'obtention, pour la première fois, d'une valve à ouverture active, c'est-à-dire une valve qui, lorsque les pressions sanguines de part et d'autre de la valve sont identiques, est légèrement ouverte.

Cette valve à ouverture active permet d'obtenir en outre un rendement hémodynamique d'environ 25 % supérieur au rendement des valves dont on dispose actuellement et permet de ce fait de diminuer les risques de coagulation du sang et d'améliorer le travail cardiaque. Enfin l'usure de la valve sera améliorée.

La valve cardiaque selon l'invention comporte un siège fixe et au moins un élément mobile assurant l'ouverture et la fermeture complète de la valve selon le sens de circulation du sang, qui exerce une pression sur l'une ou l'autre face de l'élément mobile, caractérisée en ce que ledit siège fixe et/ou ledit élément mobile comportent un dispositif, connu en soi, qui assure une ouverture déterminée de ladite valve lorsque celle-ci repose sur son siège fixe et lorsque la pression sanguine est équilibrée des deux côtés dudit élément mobile, ledit dispositif permettant néanmoins une fermeture complète de la valve lorsqu'une pression appropriée s'exerce sur celle-ci.

Ledit dispositif connu en soi peut être, de façon non limitative, de type mécanique ou magnétique. Dans les dispositifs mécaniques on utilisera par exemple une lame de ressort fixée sur le siège qui assurera une certaine poussée donc, à l'équilibre, qui provoquera une certaine ouverture de la valve. Dans les systèmes magnétiques on disposera par exemple d'au moins un petit aimant permanent sur le bord d'un clapet et d'au moins un petit aimant permanent dans le siège, ces deux aimants étant positionnés pour exercer l'un sur l'autre un effet répulsif contrôlé.

Le dispositif assurant une ouverture déterminée de la valve lorsque celle-ci est au repos est plus particulièrement utilisable dans les valves décrites dans FR-A-2 331 997. De plus, lesdites valves peuvent comporter, par rapport à ce qui est décrit dans ledit brevet, des améliorations substantielles que l'on décrit ci-après.

Ces améliorations qui peuvent être mises en oeuvre individuellement ou en combinaison sont les suivantes :

— dans FR-A-2 331 997 le basculement des clapets s'effectue autour de deux axes parallèles constitués par deux tiges cylindriques (parallèles solidaires de l'anneau ; il peut être souhaitable que ces deux axes matériels soient remplacés par deux excroissances fixées chacune sur l'anneau et formant ensemble un axe fictif positionné comme l'était celui des tiges cylindriques parallèles du précédent modèle.

— dans FR-A-2 331 997 la face externe du clapet était pourvue de picots qui encadrent la tige cylindrique formant axe et guident le déplacement en translation du clapet ; il peut être souhaitable d'éviter la présence de ces picots et d'aménager la face supérieure du clapet de façon à ce que cette face présente à la partie de cette face qui sera en contact avec l'axe matériel ou les excroissances selon l'invention, un renflement ménageant une gorge dans laquelle ledit axe matériel ou lesdites excroissances viendront s'insérer pour le pivotement dudit clapet.

L'exemple non limitatif suivant illustre l'invention ; cet exemple est illustré par les figures 1 à 5.

La figure 1 est la vue de dessus d'une des moitiés symétriques de la valve par ailleurs circulaire.

La figure 2 est la vue en coupe par un plan diamétral de la prothèse, vue représentant également ici une moitié symétrique de la prothèse.

La figure 3 représente un dispositif magnétique d'ouverture active.

Les figures 4 A et B représentent une coupe transversale d'un clapet à deux niveaux (coupe centrale 4 A et sur le bord, au niveau du renflement du clapet, coupe 4 B).

La figure 5 représente en coupe partielle un dispositif mécanique d'ouverture active.

La valve cardiaque selon l'invention comporte essentiellement deux parties : une partie fixe 1 et au moins une partie mobile 2.

La partie fixe 1 se compose d'un anneau circulaire par exemple métallique ou en carbone pyrolytique dont la face interne présente un renflement (3) sur lequel les bords du ou des clapets (2) viennent s'appuyer pour fermer la valve. Ledit renflement (3) est convenablement interrompu pour permettre le basculement des clapets. Cette partie fixe comporte également des excroissances 4 convenablement disposées ; ces excroissances (deux pour chaque clapet) constituent l'axe autour duquel le clapet pivotera. Enfin la partie fixe présente encore deux renflements (5) diamétralement opposés qui constituent un rail de butée pour limiter en arrière le déplacement des clapets et rail sur lequel la partie arrière des clapets s'appuie lors de la rotation desdits clapets.

La partie mobile (2) est constituée par deux clapets semi-circulaires en général plats et minces et de préférence constitués en carbone pyrolitique ou en matière plastique.

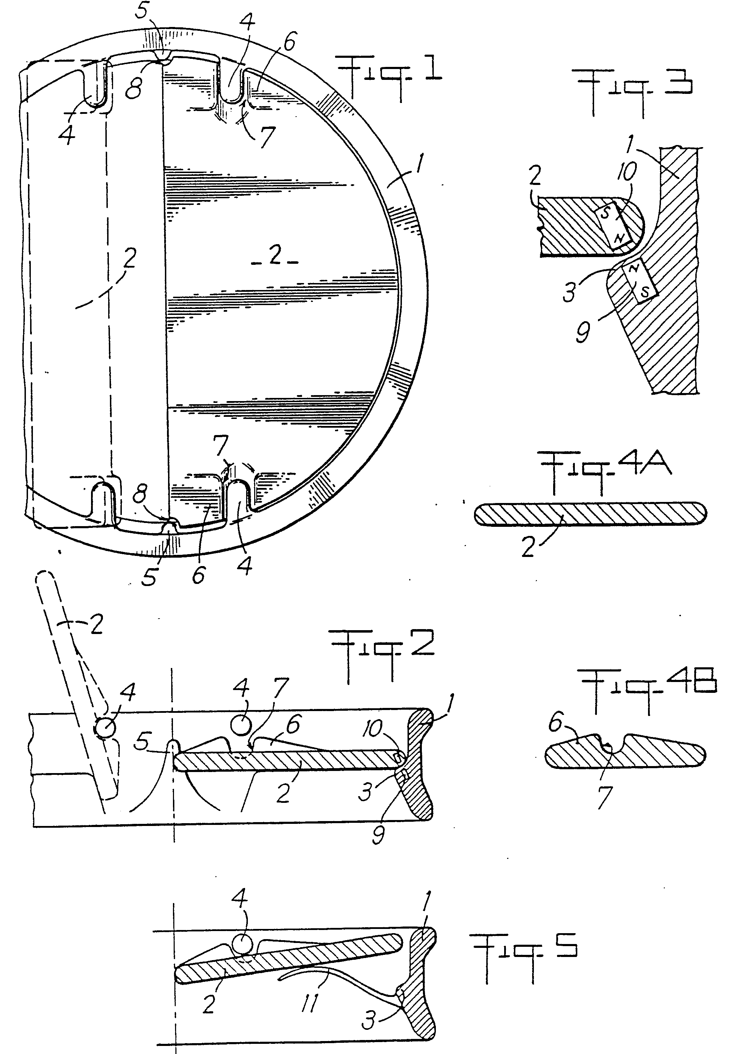

La face supérieure de chaque clapet, présente, à l'endroit où le clapet sera en contact avec les excroissances 4 deux renflements (6) dont chacun comporte une gorge 7. Lors de l'ouverture du clapet chaque excroissance 4 vient s'insérer dans ladite gorge 7 afin d'assurer ultérieurement le pivotement du clapet autour de l'axe formé par les excroissances 4-4. Lors de la fermeture du clapet le sang circulant assurera le lavage de la zone charnière constituée par les excroissances 4 et la gorge 7. Les clapets comprennent une échancrure 8 aux extrémités de leur partie linéaire qui correspond au rail de butée 5, contrôlant ainsi le déplacement en translation et rotation des clapets. La valve telle que décrite ci-dessus comporte les perfectionnements apportés aux valves décrites dans FR-A-2 331 997. Mais selon l'invention ladite valve doit également comporter au moins un dispositif qui lui assure une ouverture active. Le dispositif représenté sur la figure 3 est constitué de deux aimants disposés en regard l'un de l'autre, les pôles de même signe se faisant face, l'un de ces aimants 9 étant solidaire de l'anneau 1 formant la partie fixe de la valve et l'autre aimant 10 étant solidaire du clapet 2 de la valve ; dans la représentation de la figure 3 ces deux aimants sont noyés dans chacune des parties constitutives de la valve. Rien évidemment il peut y avoir plusieurs paires d'aimants sur le pourtour de la valve.

Les aimants (9) et/ou (10) peuvent être logés dans une surépaisseur convenable de la partie fixe et du clapet mobile ; ils peuvent également être collés à la surface de ces deux pièces ou finement dispersés (cas d'aimants microscopiques ou ultramicroscopiques) dans le matériau qui constitue la partie fixe et/ou la partie mobile de la valve.

Les divers aimants disposés sur le pourtour du clapet peuvent être remplacés par un aimant unique de forme semi-circulaire où dont la forme générale est une portion de cercle.

Il est également possible de remplacer les aimants permanents par les électroaimants ; on peut notamment utiliser la combinaison d'une pièce magnétique légère située dans la partie mobile (clapet) de la valve avec un électroaimant disposé dans la partie fixe de la valve.

Le dispositif représenté sur la figure 5 est constitué essentiellement par un ressort 11 en un métal élastique, qui est fixé à la partie fixe de la valve et qui, au repos, maintient le clapet en position légèrement ouverte.

## Revendications

1. Valve cardiaque comportant un siège fixe (1) et au moins un élément mobile (2) assurant l'ouverture et la fermeture complète de la valve selon le sens de circulation du sang, qui exerce une pression sur l'une ou l'autre face de l'élément mobile, caractérisée en ce que ledit siège fixe (1) et/ou ledit élément mobile (2) comportent un dispositif (9, 10, 11), connu en soi, qui assure une ouverture déterminée de ladite valve lorsque celle-ci repose sur son siège fixe (1) et lorsque la pression sanguine est équilibrée des deux côtés dudit élément mobile (2), ledit dispositif (9, 10, 11) permettant néanmoins une fermeture complète de la valve lorsqu'une pression appropriée s'exerce sur celle-ci.

2. Valve cardiaque selon la revendication 1, caractérisée en ce que ledit siège fixe est un anneau (1) et que ledit élément mobile est un clapet (2).

3. Valve selon l'une des revendications 1 et 2, caractérisée en ce que ledit dispositif, connu en soi, assurant la position ouverte de la valve est constituée par la combinaison de deux aimants (9) (10) à pôles opposés en vis-à-vis ou électroaimants.

4. Valve selon l'une des revendications 1 à 3, caractérisée en ce que ledit dispositif, connu en soi, est constitué par un ressorts (11).

5. Valve selon l'une des revendications 2 à 4, constituée par une partie fixe qui est un anneau circulaire (1) comportant des moyens de suture et de deux clapets (2) sensiblement semi-circulaires mobiles dont les bords reposent sur un renflement (3) dudit anneau lorsque la valve est fermée, lesdits clapets effectuant dans leurs déplacements un mouvement de translation et un mouvement de rotation, caractérisée en ce que les axes de rotation de chaque clapet sont constitués par deux excroissances (4) (4) fixées chacune sur l'anneau et formant ensemble un axe fictif, lesdites excroissances coopérant avec des gorges (7) ménagées dans deux renflements (6) convenablement disposés à la surface de chaque clapet.

## Claims

1. Cardiac valve comprising a fixed seat (1) and at least one mobile element (2) ensuring the opening and complete closure of the valve according to the direction of the blood circulation exerting a pressure on one or the other face of the mobile element, characterized in that the said fixed seat (1) and/or the said mobile element (2) comprise a device (9, 10, 11), known per se, which ensures a predetermined opening of said valve when the latter is at rest on its fixed seat (1) and when the blood pressure is balanced on both sides of said mobile element (2), said device (9,10,11) permitting, nevertheless, a complete closure of the valve when an appropriate pressure is exerted thereon.

2. The cardiac valve according to claim 1, characterized in that said fixed seat is a ring (1) and said mobile element is a check valve (2).

3. The valve according to one of claims 1 and 2, characterized in that said device, known per se, which ensures the opening of the valve, is constituted by the combination of two magnets (9) (10) with poles in opposed facing relationship or electromagnets.

4. The valve according to one of claims 1 to 3, characterized in that said device, known per se, is constituted by a spring (11).

5. The valve according to one of claims 2 to 4, constituted by a fixed part which is a circular ring (1) comprising suture means and by two mobile, substantially semi-circular check valves (2) whose edges rest on a swell (3) on said ring when the valve is closed, said check valves effecting in their displacements a movement of translation and a movement of rotation, characterized in that the axes of rotation of each check valve are constituted by two protuberances (4) (4) each fixed on the ring and together forming a fictitious axis, said protuberances cooperating with grooves (7) made in two swells (6) suitably disposed on the surface of each check valve.

## Patentansprüche

1. Herzklappe, umfassend einen feststehenden Sitz (1) und mindestens ein bewegliches Element (2), die das Öffnen und vollständige Schließen der Herzklappe im Sinne der Blutzirkulation sichert, welche einen Druck auf die eine oder die andere Seite des beweglichen Elements ausübt, dadurch gekennzeichnet, daß der feststehende Sitz (1) und/oder das bewegliche Element (2) eine an sich bekannte Einrichtung (9, 10, 11) umfassen, die ein bestimmtes Öffnen der Klappe gewährleistet, wenn diese auf ihrem feststehenden Sitz (1) ruht und wenn der Blutdruck an den beiden Seiten des beweglichen Elements (2) im Gleichgewicht ist, wobei die Einrichtung (9, 10, 11) dennoch ein vollständiges Schließen der Klappe gestattet, wenn ein entsprechender Druck auf diese ausgeübt wird.

2. Herzklappe nach Anspruch 1, dadurch gekennzeichnet, daß der feststehende Sitz ein Ring (1) und das bewegliche Element eine Ventilklappe (2) ist.

3. Herzklappe nach einem der Ansprüche 1 und 2, dadurch gekennzeichnet, daß die an sich bekannte Einrichtung zur Sicherung der geöffneten Position der Herzklappe durch die Kombination zweier Magneten (9) (10) mit einander zugewandten entgegengesetzten Polen oder Elektromagneten gebildet ist.

4. Herzklappe nach einem der Ansprüche 1 bis 3, dadurch gekennzeichnet, daß die an sich bekannte Einrichtung durch eine Feder (11) gebildet ist.

5. Herzklappe nach einem der Ansprüche 2 bis 4, gebildet durch einen feststehenden Teil, welcher ein kreisförmiger Ring (1) ist, der Nahtmittel und zwei im wesentlichen halbkreisförmige bewegliche Ventilklappen (2) umfaßt, deren Ränder auf einem Wulst (3) des Rings ruhen, wenn die Herzklappe geschlossen ist, wobei die Ventilklappen bei ihrer Verstellbewegung eine Translationsbewegung und eine Rotationsbewegung ausführen, dadurch gekennzeichnet, daß die Rotationsachsen jeder Ventilklappe durch zwei jeweils am Ring befestigte und zusammen eine fiktive Achse bildende Vorsprünge (4) (4) gebildet sind, welche Vorsprünge mit Auskehlungen (7) zusammenwirken, die in zwei entsprechend an der Oberfläche jeder Ventilklappe angeordneten Verstärkungen (6) vorgesehen sind.

Fig. 1

Fig. 3

Fig. 4A

Fig. 2

Fig. 4B

Fig. 5